# FASCICULE DE BREVET EUROPEEN

(11) **EP 2 705 139 B1**
(45) Date de publication et mention de la délivrance du brevet: **29.08.2018**
(21) Numéro de dépôt: 12722893.0
(22) Date de dépôt: 04.05.2012
(51) Int. Cl.: C12N 1/20, C12N 1/38, C12P 3/00

(54) **UTILISATION D'AU MOINS UN AGENT CHELATANT INTRODUIT DANS LE MILIEU DE CULTURE DE BACTERIES MAGNETOTACTIQUES POUR STIMULER LA CROISSANCE DE CES BACTERIES**
VERWENDUNG VON MINDESTENS EINEM IN DAS KULTURMEDIUM VON MAGNETOTAKTISCHEN BAKTERIEN EINGEBRACHTEN CHELATBILDNER ZUR STIMULIERUNG DES WACHSTUMS
USE OF AT LEAST ONE CHELATING AGENT INTRODUCED INTO THE CULTURE MEDIUM OF MAGNETOTACTIC BACTERIA IN ORDER TO STIMULATE THE GROWTH THEREOF

(30) Priorité: 06.05.2011 FR 1153938
(43) Date de publication de la demande: 12.03.2014
(73) Titulaire: Sorbonne Université, 75006 Paris (FR); Centre National de la Recherche Scientifique, 75016 Paris (FR)
(72) Inventeur: ALPHANDERY, Edouard, F-70516 Paris (FR); CHEBBI, Imène, F-93800 Epinay Sur Seine (FR)
(74) Mandataire: Nony
(86) Numéro de dépôt international: PCT/IB2012/052235
(87) Numéro de publication internationale: WO 2012/153247

(56) Documents cités:
- WO-A1-2011/061259
- MATSUNAGA T ET AL: "Mass culture of magnetic bacteria and their application to flow type immunoassays", IEEE TRANSACTIONS ON MAGNETICS 1990 SEP, vol. 26, no. 5, 17 avril 1990 (1990-04-17) , pages 1557-1559, XP002665003, DOI: DOI:10.1109/20.104444
- YANG CHEN-DONG ET AL: "Effects of growth medium composition, iron sources and atmospheric oxygen concentrations on production of luciferase-bacterial magnetic particle complex by a recombinant Magnetospirillum magneticum AMB-1", ENZYME AND MICROBIAL TECHNOLOGY, vol. 29, no. 1, 5 juillet 2001 (2001-07-05), pages 13-19, XP002665004, ISSN: 0141-0229
- ALPHANDÉRY EDOUARD ET AL: "Chains of magnetosomes extracted from AMB-1 magnetotactic bacteria for application in alternative magnetic field cancer therapy.", ACS NANO 23 AUG 2011 LNKD- PUBMED:21732678, vol. 5, no. 8, 23 août 2011 (2011-08-23), pages 6279-6296, XP002665005, ISSN: 1936-086X
- MATSUNAGA T ET AL: "MAGNETITE FORMATION BY A MAGNETIC BACTERIUM CAPABLE OF GROWING AEROBICALLY", APPLIED MICROBIOLOGY AND BIOTECHNOLOGY, SPRINGER, DE, vol. 35, no. 5, 1 January 1991 (1991-01-01), pages 651-655, XP008146083, ISSN: 0175-7598, DOI: 10.1007/BF00169632 [retrieved on 2004-11-16]
- MATSUNAGA T ET AL: "Production of luciferase-magnetic particle complex by recombinant Magnetospirillum sp. AMB-1", BIOTECHNOLOGY AND BIOENGINEERING, WILEY & SONS, HOBOKEN, NJ, US, vol. 70, no. 6, 20 December 2000 (2000-12-20), pages 704-709, XP002367587, ISSN: 0006-3592, DOI: 10.1002/1097-0290(20001220)70:6<704::AID-B IT14>3.0.CO;2-E

## Description

### DOMAINE DE L'INVENTION

La présente invention porte sur l'obtention de magnétosomes bactériens, principalement sous forme de chaînes de magnétosomes extraites des bactéries magnétotactiques après la culture de ces bactéries. Plus particulièrement, l'invention porte sur l'utilisation d'additifs spécifiques, préférentiellement des agents chélatants du fer, qui sont introduits dans le milieu de culture des bactéries magnétotactiques et permettent de stimuler la croissance de ces bactéries. Cela a pour conséquence d'augmenter leur rendement de production au cours du temps. Ces chaînes de magnétosomes peuvent par exemple être utilisées dans le domaine de la thérapie des cancers ou dans le domaine du diagnostic des cancers.

### ART ANTERIEUR

Les magnétosomes sont des nanocristaux mono-domaines recouverts d'une membrane phospholipidique, composés de magnétite ou greigite. Les magnétosomes peuvent s'oxyder suite à leur extraction des bactéries passant d'une composition de magnétite à une composition de maghémite. Les magnétosomes sont habituellement organisés sous forme de chaînes dans les bactéries. Les bactéries magnétotactiques utilisent ces chaînes de magnétosomes comme d'un compas pour naviguer dans la direction du champ magnétique terrestre, ce qui leur permet de trouver les conditions optimales nécessaires à leur survie et à leur développement (Bazylinski et al., Nat. Rev. Microbiol. 2004, 2, 217-230). On a montré que les magnétosomes peuvent être utiles pour un nombre important d'applications tant dans les domaines scientifiques que commerciaux ou médicaux. Par exemple, ils peuvent être utilisés pour détecter des polymorphismes de nucléotides individuels, pour extraire l'ADN, pour détecter magnétiquement les interactions biomoléculaires. Ils peuvent aussi être utilisés dans des essais immunologiques ou d'accrochage de récepteur ou pour séparer les cellules (Arakaki et al., J. R. Soc. Interface, 2005, 5, 977-999). Il a été suggéré que les magnétosomes bactériens pouvaient être insérés dans des liposomes pour délivrer des médicaments (Brevet US 625,1365 B1). L'activité anti-tumorale d'un complexe formé des magnétosomes bactériens et de la doxorubicine a également été montrée expérimentalement (Sun et al., Cancer. Lett., 2007, 258, 109-117).

De plus, des études récentes semblent indiquer que les magnétosomes sont peu toxiques, ce qui en fait de bons candidats pour des applications médicales (Sun et al., J. Nanosci. Nanotechnol., 2009, 9, 1881-1885, Sun et al., Nanotoxicology, 2010, 4, 271-283).

Dans le domaine d'application sur lequel les inventeurs travaillent, c'est-à-dire l'hyperthermie magnétique, il a été montré que les magnétosomes ont une grande efficacité. L'hyperthermie magnétique est une technique utilisée généralement pour les traitements de cancer (tumeurs solides) suivant laquelle des nanoparticules magnétiques sont envoyées ou administrées dans les tumeurs puis chauffées sous application d'un champ magnétique alternatif. La chaleur dégagée par les nanoparticules produit l'effet anti-tumoral, la destruction des cellules tumorales. Du fait de leur importante capacité de chauffage (qui est du essentiellement à leur grande taille) et de leur arrangement en chaînes, les inventeurs ont montré que les magnétosomes sont particulièrement efficaces pour l'hyperthermie magnétique (Alphandéry et al., J. Phys. Chem. C, 2011, 115, 18-22, brevet PCT/EP2010/067765). Les inventeurs ont également montré dans le brevet PCT/EP2010/067765 que les chaînes de magnétosomes sont plus efficaces que les nanoparticules d'oxyde de fer superparamagnétiques (SPION) qui sont actuellement utilisées pour l'hyperthermie magnétiques (PCT n° WO 2004/064921 et demandes de brevet américain n° US 2003/0028071, n° US 2006/0167313 et n° US 2008/0268061). Les résultats expérimentaux déjà obtenus indiquent que les chaînes de magnétosomes sont de bons candidats pour l'hyperthermie magnétique.

Les magnétosomes peuvent donc être utilisés pour de nombreuses applications étant donné leurs propriétés avantageuses ou différentes de celles des nanoparticules synthétisées par voie chimique (les SPION). Cependant, le faible rendement de production des bactéries magnétotactiques est une difficulté qu'il reste à surmonter afin de permettre une commercialisation aisée de ces magnétosomes. Des efforts ont été entrepris pour améliorer le rendement de production des bactéries magnétotactiques notamment en proposant de réaliser la culture des bactéries magnétotactiques dans des conditions contrôlées (U. Heyen et al., Appl. Microbiol. Biotechnol., 2003, 61, 536-544, C. Lang et al., J. Phys. Condens. Matter., 2006, 18, S2815-S2828, brevets Chinois CN 101376900 (A), CN 101434922 (A), CN 101434921 (A)). Matsunaga et al. (Biotechnol. Bioeng. 2000, 70(6):704-709) décrit que l'ajout en continu de quinate de fer augmente la croissance de Magnetospirillium sp. AMB-1.

### RESUME DE L'INVENTION

Les inventeurs ont recherché la mise au point de procédés permettant la production de magnétosomes avec un rendement amélioré.

Dans ce contexte, la présente invention propose d'utiliser des additifs variés pour stimuler la croissance des bactéries magnétotactiques, ce qui a pour conséquence d'augmenter le rendement de production des bactéries magnétotactiques au cours du temps.

La méthode proposée selon l'invention peut être combinée aux méthodes déjà connues dans l'état de la technique pour améliorer encore plus le rendement de production des bactéries magnétotactiques, dont certaines ont été citées ci-dessus.

La présente invention concerne l'utilisation d'au moins un agent consistant en un agent chélatant, y compris un agent chélatant du fer, pour stimuler la croissance de bactéries magnétotactiques.

Dans certains modes de réalisation, les agents chélatants sont choisis notamment parmi (i) les agents chélatants possédant un ou plusieurs groupes carboxyles, (ii) les agents chélatants possédant un ou plusieurs groupes hydroxyles, (iii) les agents chélatants possédant un ou plusieurs groupes amino et/ou carboxyles et/ou cétones, (iv) les agents chélatants possédant un ou plusieurs groupes phosphonates et/ou acide phosphoniques, (v) les agents chélatants possédant un ou plusieurs groupes bis-phosphonates et/ou tris-phosphonates et/ou tétra-phosphonates, (vi) les agents chélatants possédant un ou plusieurs groupes sulfonates et/ou acide sulfoniques et (vii) les agents chélatants de type polydentés, du type polymérique par exemple du type polysaccharidique.

Dans certains modes de réalisation, ledit agent chélatant est choisi parmi la rhodamine B, l'acide ascorbique, l'acide citrique, l'acide folique, l'érythrosine, l'hémoglobine, un dextran de bas poids moléculaire, l'acide anthranilique, la calcéine, l'alendronate, l'acide 3-[cyclohexylamino]-1-propanesulfonique (CAPS) et l'EDTA.

### DESCRIPTION DES FIGURES

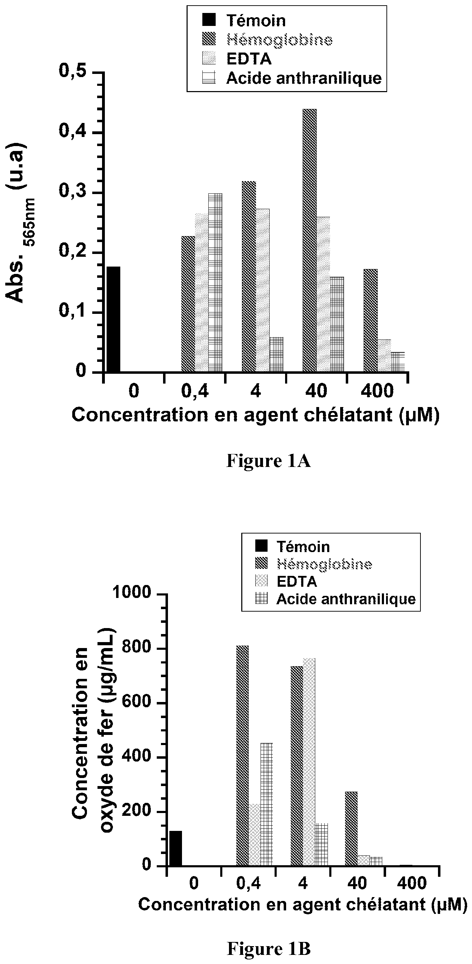

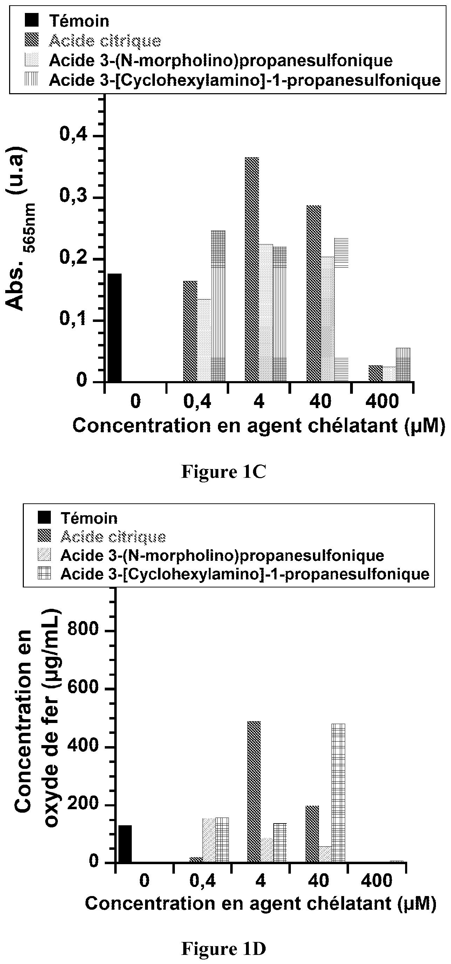

La **Figure 1** illustre l'effet de l'hémoglobine, de l'EDTA, de l'acide anthranilique, de l'acide citrique, de l'acide 3-(N-morpholino)propanesulfonique et de l'acide 3-[Cyclohexylamino]-1-propanesulfonique sur la croissance des bactéries magnétotactiques et sur la production des magnétosomes au bout de 7 jours de culture. Cette étude a été réalisée sur 1 litre de milieu de culture.
La ***Figure 1A*** représente, sous forme d'histogramme, l'absorption (mesurée à 565 nm) en fonction de la concentration en hémoglobine, EDTA et acide anthranilique. En ordonnées : absorbance, exprimée en unités arbitraires (u.a). En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : hémoglobine, EDTA et acide anthranilique.
La ***Figure 1B*** représente, sous forme d'histogramme, la concentration en oxyde de fer (mesurée par absorption à 480 nm) en fonction de la concentration en hémoglobine, EDTA et acide anthranilique. En ordonnées : concentration en oxyde de fer, exprimée en µg/mL En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : hémoglobine, EDTA et acide anthranilique.
La ***Figure 1C*** représente, sous forme d'histogramme, l'absorption (mesurée à 565 nm) en fonction de la concentration en acide citrique, acide 3-(N-morpholino) propanesulfonique et acide 3-[Cyclohexylamino]-1-propanesulfonique. En ordonnées : absorbance, exprimée en unités arbitraires (u.a). En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : acide citrique, acide 3-(N-morpholino) propanesulfonique et acide 3-[cyclohexylamino]-1-propanesulfonique.
La ***Figure 1D*** représente, sous forme d'histogramme, la concentration en oxyde de fer (mesurée par absorption à 480 nm) en fonction de la concentration en acide citrique, acide 3-(N-morpholino) propanesulfonique et acide 3-[Cyclohexylamino]-1-propanesulfonique. En ordonnées : concentration en oxyde de fer, exprimée en µg/mL. En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : acide citrique, acide 3-(N-morpholino)propanesulfonique et acide 3-[cyclohexylamino]-1-propanesulfonique.

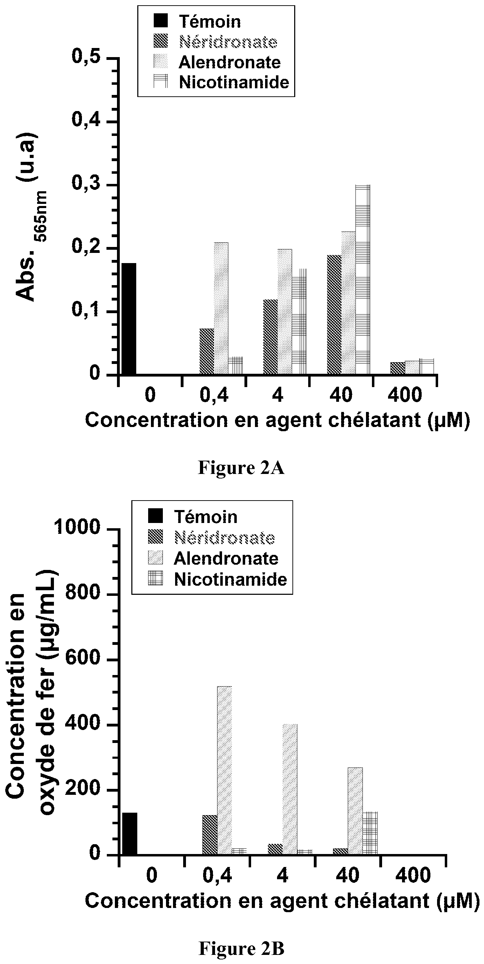

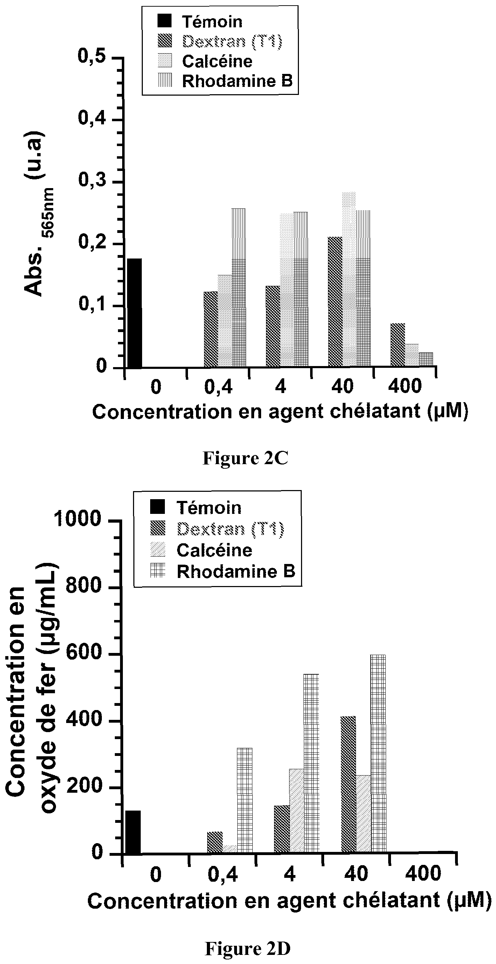

La **Figure 2** illustre l'effet du néridronate, alendronate, nicotinamide, dextran, calcéine et rhodamine B sur la croissance des bactéries magnétotactiques et sur la production des magnétosomes au bout de 7 jours de culture. Cette étude a été réalisée sur 1 litre de milieu de culture.
La ***Figure 2A*** représente, sous forme d'histogramme, l'absorption (mesurée à 565 nm) en fonction de la concentration en Néridronate, Alendronate et Nicotinamide. En ordonnées : absorbance, exprimée en unités arbitraires (u.a). En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : néridronate, alendronate et nicotinamide.
La ***Figure 2B*** représente, sous forme d'histogramme, la concentration en oxyde de fer (mesurée par absorption à 480 nm) en fonction de la concentration en Néridronate, Alendronate et Nicotinamide. En ordonnées : concentration en oxyde de fer, exprimée en µg/mL En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : néridronate, alendronate et nicotinamide.
La ***Figure 2C*** représente, sous forme d'histogramme, l'absorption (mesurée à 565 nm) en fonction de la concentration en dextran (T1), calcéine et rhodamine B. En ordonnées : absorbance, exprimée en unités arbitraires (u.a). En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : dextran (T1), calcéine et rhodamine.
La ***Figure 2D*** représente, sous forme d'histogramme, la concentration en oxyde de fer (mesurée par absorption à 480 nm) en fonction de la concentration en Dextran, Calcéine et Rhodamine B. En ordonnées : concentration en oxyde de fer, exprimée en µg/mL. En abscisse : concentration de l'agent chélatant. De gauche à droite de la figure : le témoin en l'absence d'agent chélatant, puis en présence de l'agent chélatant aux concentrations croissantes de 0,4 µM, 4 µM, 40 µM et 400 µM. De gauche à droite, pour chaque concentration d'agent chélatant : dextran (T1), calcéine et rhodamine.

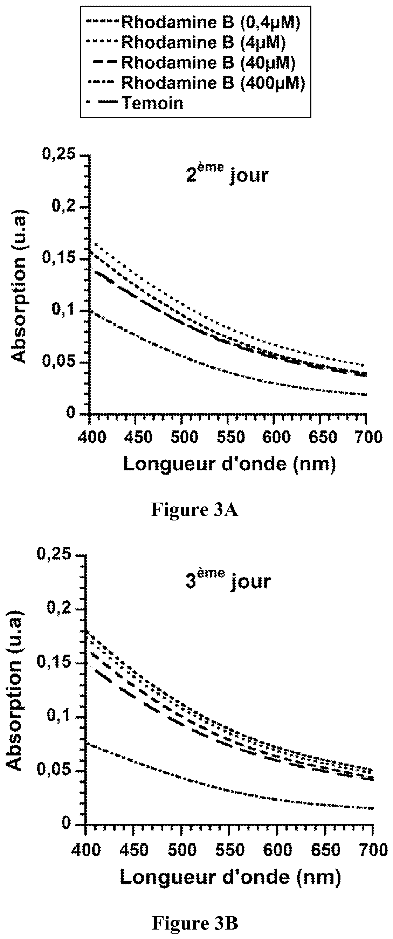

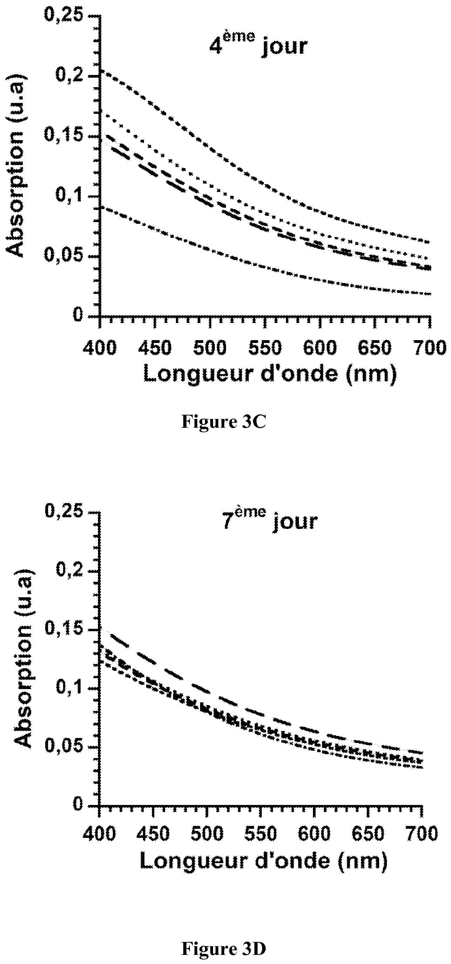

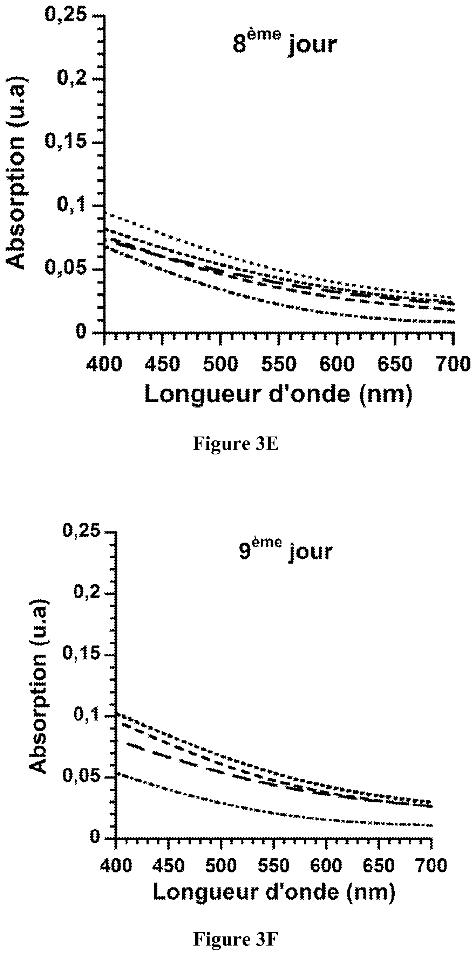

La **Figure 3** montre les spectres d'absorption des bactéries magnétotactiques cultivées en présence de différentes concentrations de Rhodamine B (0,4 µM, 4 µM, 40 µM et 400 µM) au deuxième jour, (3A), troisième jour, (3B), quatrième jour, (3C), septième jour, (3D), huitième jour, (3E), et neuvième jour, (3F), après l'inoculation de 100 µl de bactéries dans des tubes contenant 10 ml de milieu de culture. En ordonnées : valeurs d'absorbance, exprimées en unités arbitraires (u.a). En abscisse : longueur d'onde, exprimée en nanomètres.

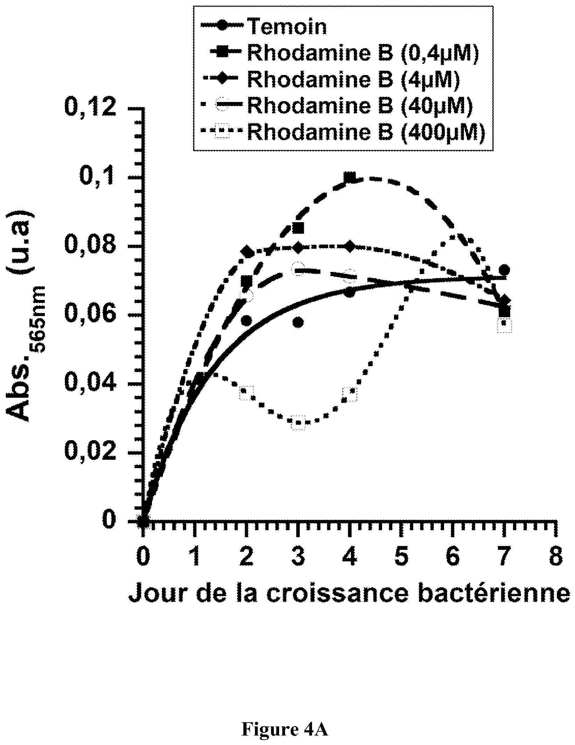

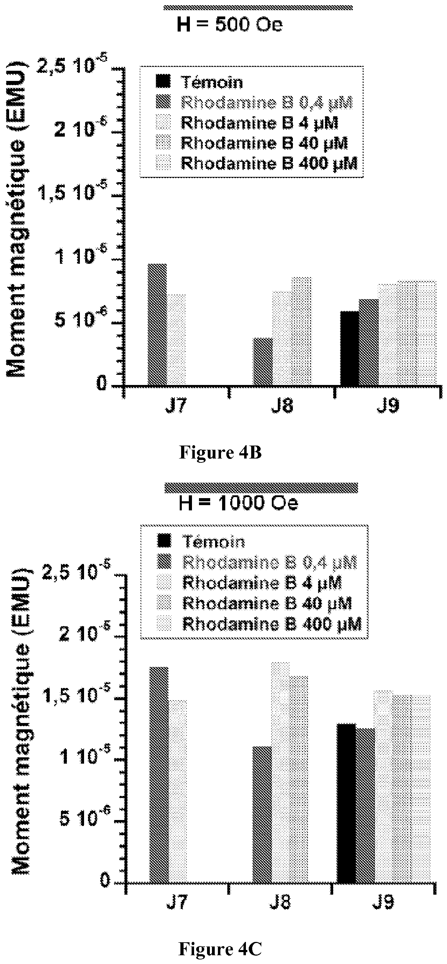

La **Figure 4** illustre l'effet de la présence de la Rhodamine B dans le milieu de culture des bactéries magnétotactiques sur la prolifération bactérienne et sur la production des magnétosomes.
La ***Figure 4A*** montre la variation de l'absorption en fonction du jour de croissance des bactéries dans un milieu contenant différentes concentrations de Rhodamine B. En ordonnées : valeurs d'absorbance à 565 nanomètres, exprimée en unités arbitraires (u.a). En abscisse : durée de la culture de bactéries, exprimée en jours.
La ***Figure 4B*** montre la variation du moment magnétique mesurée à 500 Oe à l'aide du SQUID, MPMS-5S au septième jour (J7), huitième jour (J8) et neuvième jour (J9) suivant l'inoculation de 100 µl de bactéries dans des tubes de 10 ml contenant du milieu de culture avec des concentrations croissantes de Rhodamine B de 0,4 µM, 4 µM, 40 µM et 400 µM. En ordonnées : valeur du moment magnétique, exprimée en unités EMU. En abscisse : durée de culture, exprimée en jours.
La ***Figure 4C*** montre la variation du moment magnétique mesurée à 1000 Oe à l'aide du SQUID, MPMS-5S au septième jour (J7), huitième jour (J8) et neuvième jour (J9) suivant l'inoculation de 100 µl de bactéries dans des tubes de 10 ml contenant du milieu de culture avec des concentrations croissantes de Rhodamine de 0,4 µM, 4 µM, 40 µM et 400 µM. En ordonnées : valeur de moment magnétique, exprimée en unités EMU. En abscisse : durée de culture, exprimée en jours.

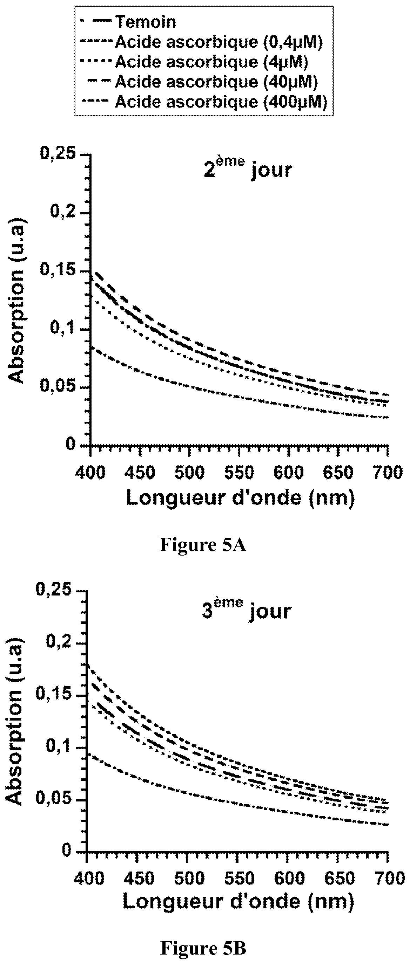

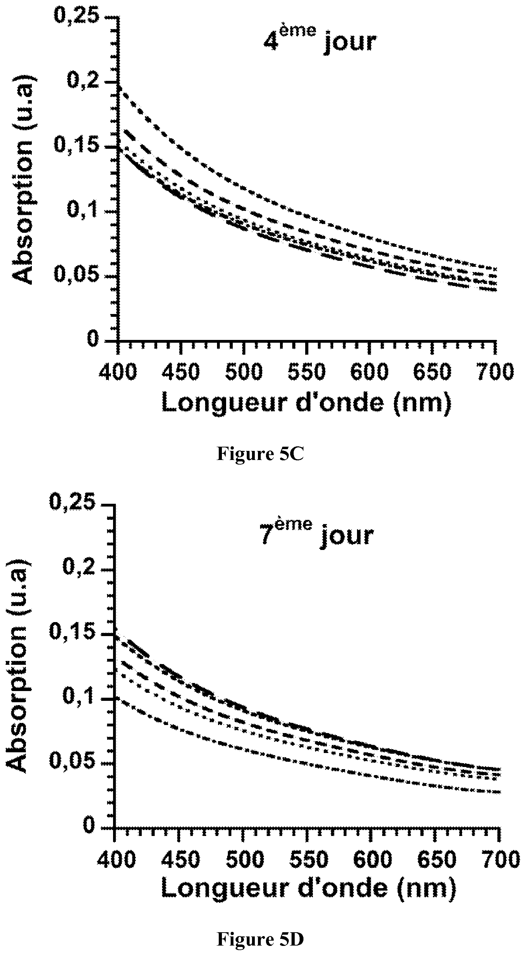

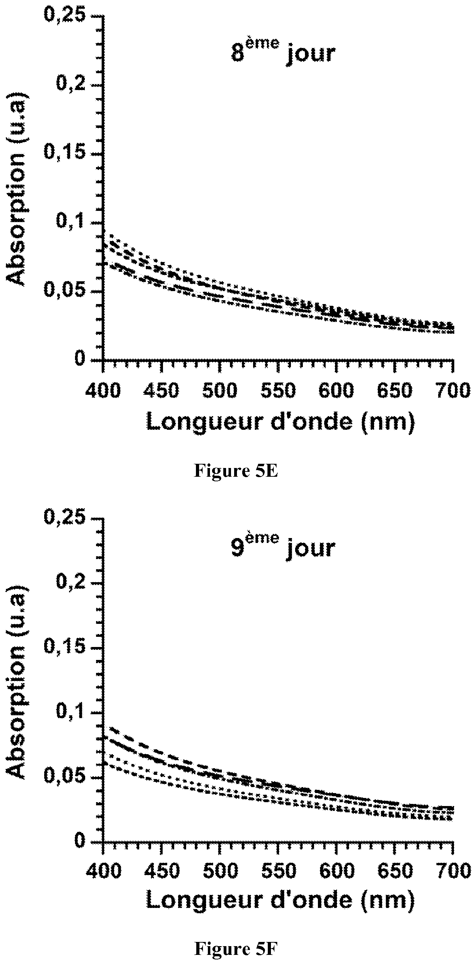

La **Figure 5** montre les spectres d'absorption des bactéries magnétotactiques cultivées en présence de différentes concentrations d'acide ascorbique (0,4 µM, 4 µM, 40 µM et 400 µM) au deuxième jour, (5A), troisième jour, (5B), quatrième jour, (5C), septième jour, (5D), huitième jour, (5E), et neuvième jour, (5F), après l'inoculation de 100 µl de bactéries dans des tubes contenant 10 ml de milieu de culture. En ordonnées : valeurs d'absorbance, exprimées en unités arbitraires (u.a). En abscisse : longueur d'onde, exprimée en nanomètres.

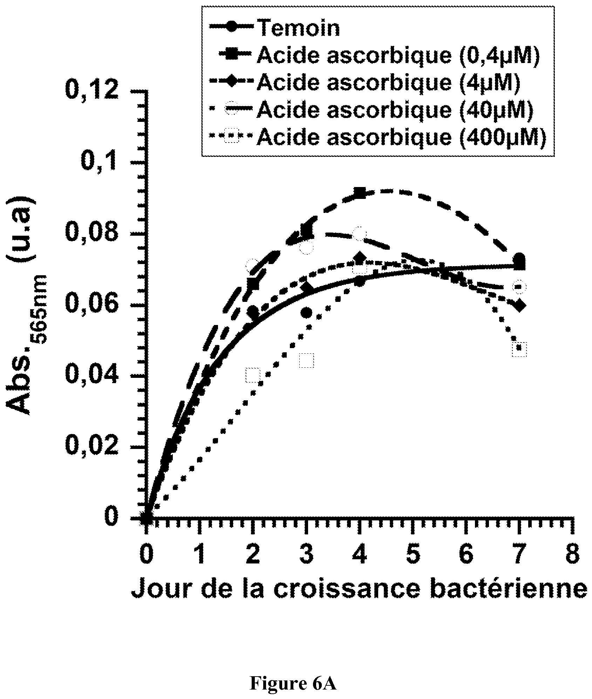

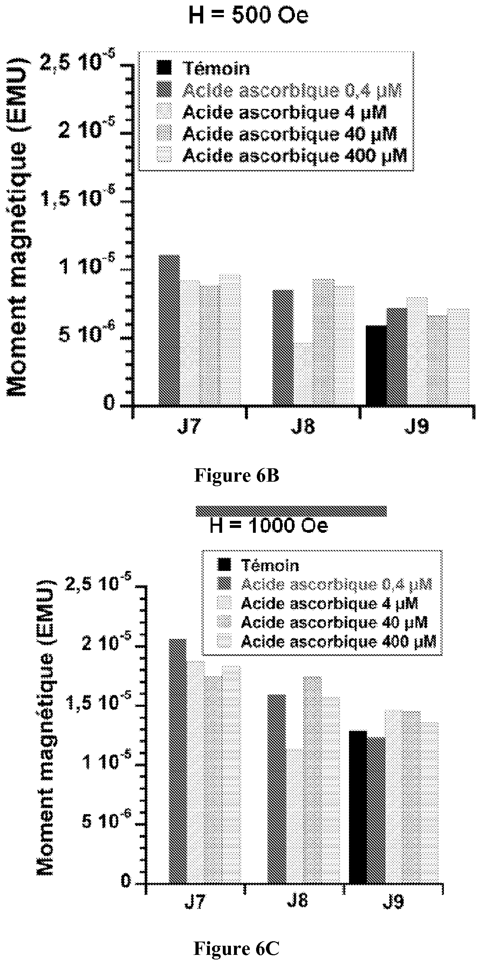

La **Figure 6** illustre l'effet de la présence de l'acide ascorbique dans le milieu de culture des bactéries magnétotactiques sur la prolifération bactérienne et sur la production de magnétosomes.
La ***Figure 6A*** montre la variation de l'absorption en fonction du jour de croissance des bactéries dans un milieu contenant des concentrations croissantes d'acide ascorbique de 0,4 µM, 4 µM, 40 µM et 400 µM. En ordonnées : valeurs d'absorbance à 565 nanomètres, exprimée en unités arbitraires (u.a). En abscisse : durée de la culture de bactéries, exprimée en jours.
La ***Figure 6B*** montre la variation du moment magnétique mesurée à 500 Oe à l'aide du SQUID, MPMS-5S au septième jour (J7), huitième jour (J8) et neuvième jour (J9) suivant l'inoculation de 100 µl de bactéries dans des tubes de 10 ml contenant du milieu de culture avec des concentrations croissantes d'acide ascorbique de 0,4 µM, 4 µM, 40 µM et 400 µM. En ordonnées : valeur de moment magnétique, exprimée en unités EMU. En abscisse : durée de culture, exprimée en jours.
La ***Figure 6C*** montre la variation du moment magnétique mesurée à 1000 Oe à l'aide du SQUID, MPMS-5S au septième jour (J7), huitième jour (J8) et neuvième jour (J9) suivant l'inoculation de 100 µl de bactéries dans des tubes de 10 ml contenant du milieu de culture avec des concentrations croissantes d'acide ascorbique de 0,4 µM, 4 µM, 40 µM et 400 µM. En ordonnées : valeur de moment magnétique, exprimée en unités EMU. En abscisse : durée de culture, exprimée en jours.

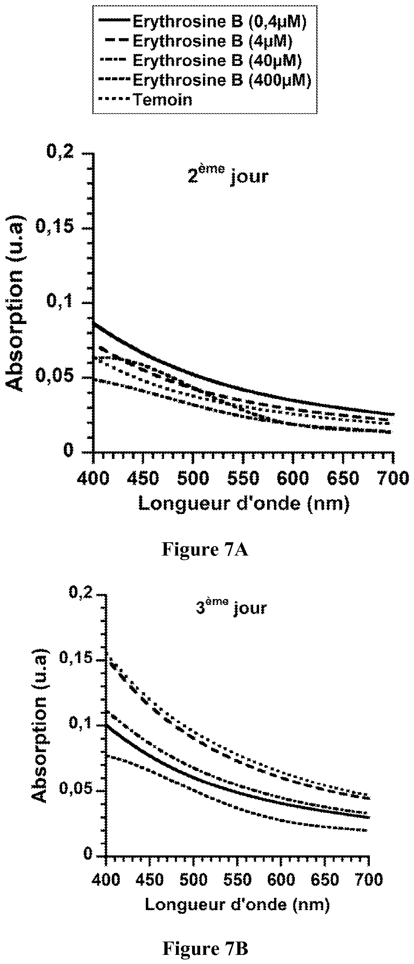

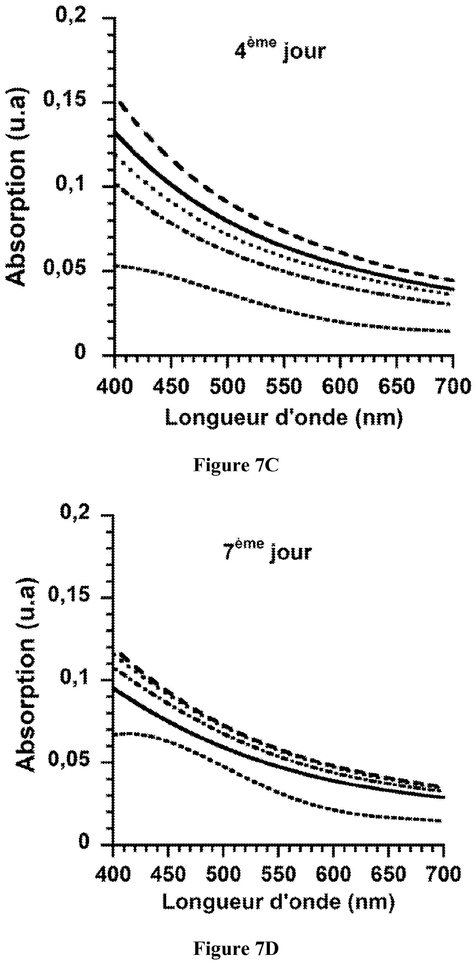

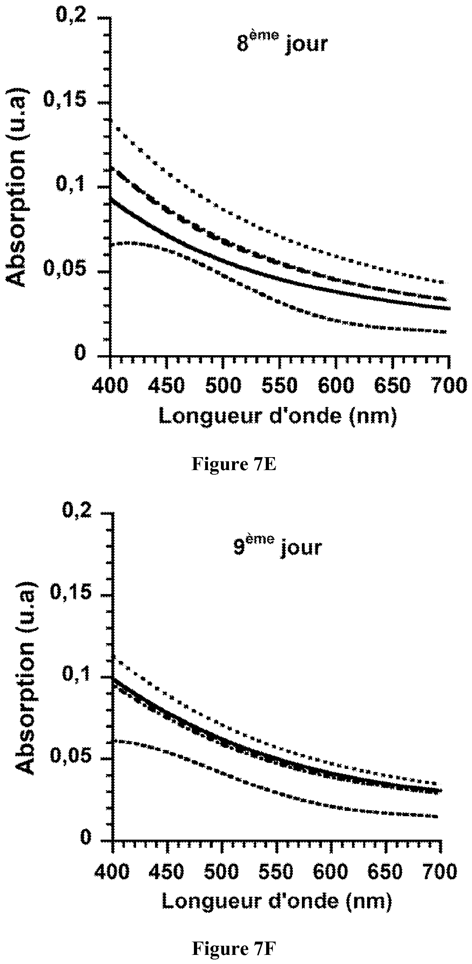

La **Figure 7** montre les spectres d'absorption des bactéries magnétotactiques cultivées en présence de différentes concentrations d'Erythrosine (0,4 µM, 4 µM, 40 µM et 400 µM) au deuxième jour, (7A), troisième jour, (7B), quatrième jour, (7C), septième jour, (7D), huitième jour, (7E), et neuvième jour, (7F), après l'inoculation de 100 µl de bactéries dans des tubes contenant 10 ml de milieu de culture. En ordonnées : valeurs d'absorbance, exprimées en unités arbitraires (u.a). En abscisse : longueur d'onde, exprimée en nanomètres.

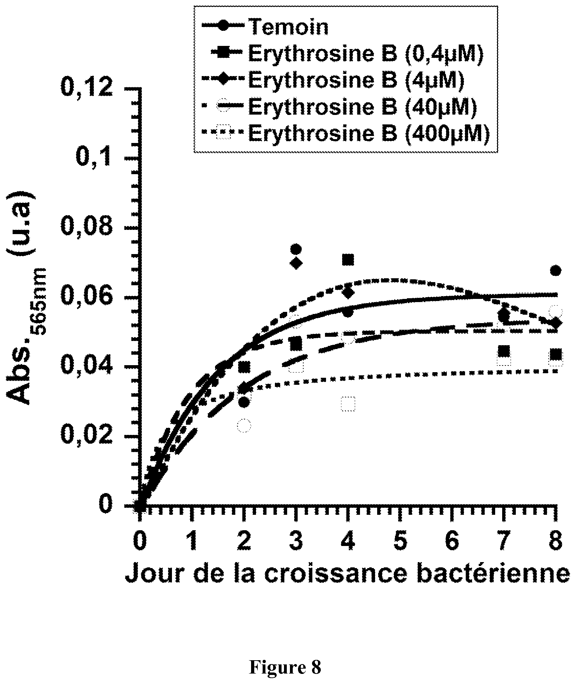

La **Figure 8** montre la variation de l'absorption en fonction du jour de croissance des bactéries magnétotactiques dans un milieu contenant des concentrations croissantes d'érythrosine de 0,4 µM, 4 µM, 40 µM et 400 µM. En ordonnées : valeurs d'absorbance à 565 nanomètres, exprimée en unités arbitraires (ua). En abscisse : durée de la culture de bactéries, exprimée en jours.

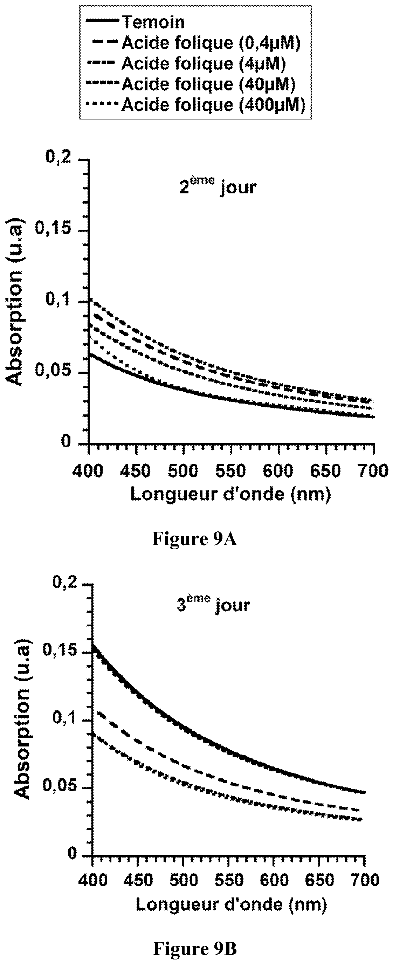

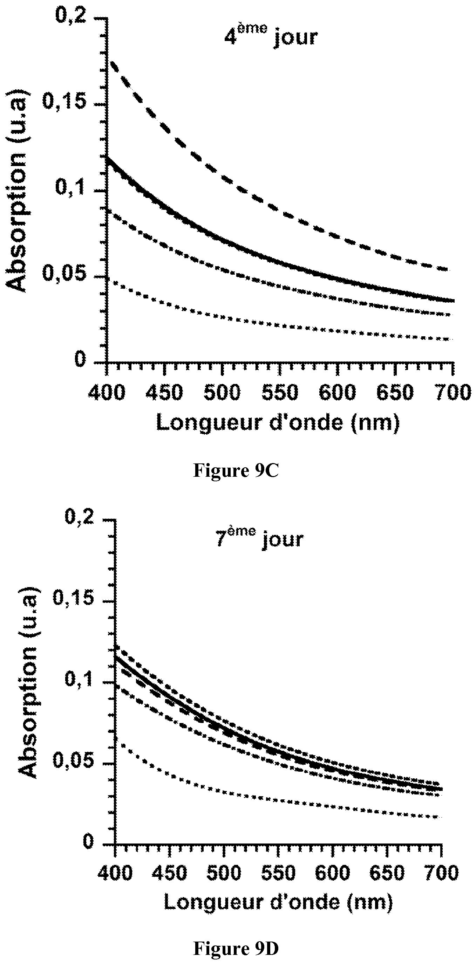

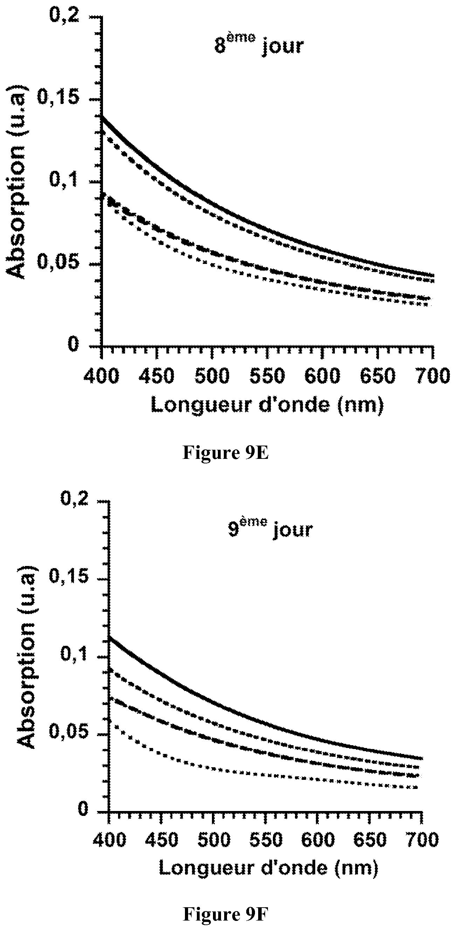

La **Figure 9** montre les spectres d'absorption des bactéries magnétotactiques cultivées en présence de différentes concentrations d'acide folique (0,4 µM, 4 µM, 40 µM et 400 µM) au deuxième jour, (9A), troisième jour, (9B), quatrième jour, (9C), septième jour, (9D), huitième jour, (9E), et neuvième jour, (9F), après l'inoculation de 100 µl de bactéries dans des tubes contenant 10 ml de milieu de culture. En ordonnées : valeurs d'absorbance, exprimées en unités arbitraires (u.a). En abscisse : longueur d'onde, exprimée en nanomètres.

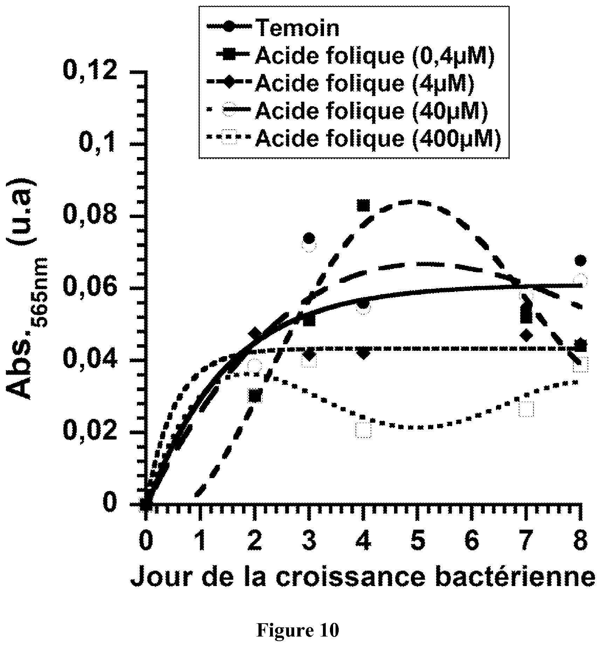

La **Figure 10** montre la variation de l'absorption en fonction du jour de croissance des bactéries magnétotactiques dans un milieu contenant des concentrations croissantes d'acide folique de 0,4 µM, 4 µM, 40 µM et 400 µM. En ordonnées : valeurs d'absorbance à 565 nanomètres, exprimée en unités arbitraires (u.a). En abscisse : durée de la culture de bactéries, exprimée en jours.

### DESCRIPTION DETAILLEE DE L'INVENTION

Il est fourni selon l'invention de nouveaux procédés d'obtention de chaînes de magnétosomes bactériens.

On a montré selon l'invention que la culture de bactéries magnétotactiques en présence d'au moins un agent consistant en un agent chélatant, en particulier d'au moins un agent chélatant du fer, stimule significativement la croissance des dites bactéries et permet ainsi l'obtention d'une quantité accrue de cellules bactériennes après une durée donnée de culture.

L'effet de stimulation de la croissance de bactéries magnétotactiques a été montré dans les exemples avec une grande variété d'agents chélatants, en particulier avec une grande variété d'agents chélatant du fer, tels que :
- un agent chélatant comprenant un ou plusieurs groupes carboxyle, tels que l'EDTA, la rhodamine B, l'acide ascorbique, l'acide anthranilique, la calcéine, l'érythrosine ou l'acide 3-[cyclohexylamino]-1-propanesulfonique (CAPS),
- un agent chélatant comprenant un ou plusieurs groupes de type phosphonates, y compris bis-phosphonates, tris-phosphonates ou tétra-phosphonates, tel que l'alendronate et le néridronate,
- un agent chélatant polymère, tel qu'un dextrane de bas poids moléculaire (poids moléculaire allant de 500 Da à 2000 Da, comme le dextran Tl), ou un carboxymethyl-dextran de bas poids moléculaire (poids moléculaire allant de 500 Da à 2000 Da), et
- un agent chélatant de type polydentate, tel que l'hémoglobine.

La présente invention concerne l'utilisation, d'au moins un agent consistant en un agent chélatant, de préférence d'au moins un agent chélatant du fer, pour stimuler la croissance de bactéries magnétotactiques.

Comme il ressort des données figurant ci-après, une stimulation de la croissance des bactéries magnétotactiques est à distinguer de l'augmentation de la production des magnétosomes par ces mêmes bactéries, qui peut être conjointement observée. La stimulation de la croissance des bactéries se traduit avant tout par une accélération du processus de maturation de ces bactéries.

L'ensemble des résultats obtenus par le demandeur montrent que l'utilisation d'un agent consistant en un agent chélatant, ou d'une combinaison d'agents chélatants, dans une culture de bactéries magnétotactiques, rend possible une obtention d'une biomasse bactérienne comprenant des chaînes de magnétosomes soit (i) plus rapidement que lorsque lesdites bactéries magnétotactiques sont cultivées en l'absence dudit ou desdits agent(s) chélatant(s) soit (ii) en plus grande quantité que lorsque lesdites bactéries magnétotactiques sont cultivées en l'absence dudit ou desdits agent(s) chélatant(s) soit (iii) plus rapidement et en plus grande quantité que lorsque lesdites bactéries magnétotactiques sont cultivées en l'absence dudit ou desdits agent(s) chélatant(s).

Par « bactérie magnétotactique », on entend selon l'invention une bactérie qui synthétise des magnétosomes qui sont généralement arrangés en chaînes tel que cela est décrit dans l'état de l'art. La spécificité de ces bactéries est qu'elles utilisent ces chaînes de magnétosomes comme d'un compas pour naviguer dans la direction du champ magnétique terrestre. L'invention se rapporte à ce type de bactérie. Les bactéries magnétotactiques englobent *Magnetospitillum magneticum* (p.ex. les souches AMB-1 et MS-1, et la souche anaérobie facultative MGT-1) la souche de *Magentococcus* MC-1, les souches de *Vibrio* anaérobies facultatives MV-1, MV-2 et MV-4, *Magnetospirillum gryphiswaldense* (p. ex. la souche MSR-1), ainsi que *Desulfovibrio magneticus* (p. ex. la souche RS-1). D'autres souches bactériennes qui peuvent être assimilées à des bactéries magnétotactiques sont également englobées par l'invention.

Par agent chélatant, on entend selon l'invention un agent apte à complexer au moins un métal de transition préférentiellement choisi parmi le fer, le cuivre, le zinc, le manganèse, le cobalt et le nickel. Les agents chélatants préférés selon l'invention sont les agents chélatants du fer.

La croissance de bactéries dans un milieu de culture non renouvelé comporte généralement plusieurs phases, respectivement (i) une phase de latence, (ii) une phase d'accélération, (iii) une phase exponentielle, (iv) une phase de décélération et (v) une phase stationnaire. Au cours de la phase de latence, (i), les bactéries synthétisent habituellement les enzymes qui leur seront nécessaires pour utiliser les substrats. Au cours de la phase d'accélération (ii), les divisions bactériennes commencent. Au cours de la phase exponentielle (iii), la vitesse de multiplication des cellules bactériennes est maximale. Au cours de la phase de décélération (iv), la vitesse de multiplication des cellules bactériennes décroit, en raison notamment de l'épuisement croissant des ressources en nutriments. Enfin, au cours de la phase stationnaire (v), la vitesse de multiplication devient nulle, avec autant de cellules qui naissent que de cellules qui meurent. On peut ajouter que, si la culture est poursuivie, la phase stationnaire est suivie d'une phase de déclin où les cellules meurent sans être renouvelées, du fait du manque de nutriments.

Par « stimulation de la croissance » de bactéries magnétotactiques, on entend un accroissement de la vitesse à laquelle lesdites bactéries se multiplient ou de la quantité de bactéries magnétotactiques produite. Cette stimulation peut être initiée au cours de n'importe quelle phase de la croissance bactérienne. Elle intervient plus particulièrement au cours de la phase exponentielle. Ainsi, pour une culture de bactéries magnétotactiques réalisée en présence d'un agent chélatant, ou d'une combinaison d'agents chélatants, ladite culture étant dans n'importe laquelle des phases (i) à (v), plus particulièrement dans la phase de croissance exponentielle, une stimulation de la croissance des bactéries est mesurée soit (i) lorsque la quantité de bactéries cultivées en présence d'agents(s) chélatant(s) est supérieure à la quantité de bactéries d'une culture identique mais réalisée en l'absence dudit agent chélatant, ou de ladite combinaison d'agents chélatants soit (ii) lorsque l'augmentation mesurée au cours du temps du nombre de bactéries cultivées en présence d'agents(s) chélatant(s) est plus rapide que l'augmentation mesurée au cours du temps du nombre de bactéries d'une culture identique mais réalisée en l'absence dudit agent chélatant, ou de ladite combinaison d'agents chélatants.

La quantité de bactéries présentes en suspension dans un milieu de culture est proportionnelle à la valeur de l'absorption de cette suspension. Plus l'absorption est importante, plus le nombre de bactéries présentes dans la suspension est important. Il est ainsi d'usage courant de mesurer la variation du nombre de bactéries en fonction du jour de croissance, ce qui est appelée une courbe de croissance, par spectrophotométrie, en mesurant l'absorbance à au moins une longueur d'onde donnée en fonction du jour de croissance (U. Heyen, Appl. Microbiol. Biotechnol. 2003, 61, 536-544). Dans certains modes de réalisation, l'absorbance est mesurée à une pluralité de longueurs d'ondes, y compris à deux longueurs d'ondes données. Un spectre allant du proche ultraviolet au proche infrarouge a été effectué, à savoir de 300 nanomètres à 600 nanomètres, avec une vitesse de scan de 100 nm/min et un intervalle de mesure de 0,5 nm entre chaque mesure d'absorbance. Dans certains modes de réalisation, par exemple lorsqu'on utilise un agent chélatant absorbant la lumière à la longueur d'onde de mesure, la mesure d'absorbance peut être réalisée à au moins une autre longueur d'onde à laquelle ledit agent chélatant n'absorbe pas la lumière. Toutefois, dans la plupart des cas, la faible quantité d'agent chélatant qui est ajoutée pour stimuler la croissance des bactéries magnétotactiques n'entraîne pas de changement important dans les propriétés d'absorption de la lumière de la suspension cellulaire, à la longueur d'onde de mesure.

Au sens de l'invention, la croissance des bactéries magnétotactiques est stimulée lorsque la culture étudiée présente une valeur d'absorbance supérieure d'au moins 5 %, par rapport à une culture témoin. En général, l'absorbance est classiquement exprimée en valeur de densité optique (D.O.), comme cela est notamment illustré dans les exemples.

Une valeur de D.O supérieure d'au moins 5 % par rapport à un témoin englobe des valeurs de D.O. supérieures d'au moins 10 %, 15 %, 20 % 25 %, 30 %, 35 %, 40 %, 45 %, 50 %, 55 %, 60 %, 65 %, 70 %, 75 %, 80 %, 90 %, 95 %, ou au moins 100 %, par rapport audit témoin.

Selon l'invention, la stimulation de la croissance des bactéries magnétotactiques en présence d'un ou de plusieurs agents chélatants introduits dans le milieu de culture est en général accompagnée d'une augmentation de la quantité de magnétosomes produite. La production de magnétosomes en plus grande quantité en présence d'un agent chélatant peut résulter de l'effet de stimulation de la croissance des bactéries magnétotactiques qui a été identifié selon l'invention. La production de magnétosomes en plus grande quantité peut aussi résulter d'une production accrue de magnétosomes par chaque cellule bactérienne ou bien encore d'un pourcentage accru de cellules bactériennes produisant des magnétosomes. La production accrue de magnétosomes peut aussi résulter d'une combinaison des mécanismes précités.

L'utilisation d'un agent chélatant, ou d'une combinaison d'agents chélatants, dans un procédé de culture de bactéries magnétotactiques permet aussi, pour un nombre donné de bactéries dans une culture de départ, l'obtention plus rapide d'une quantité donnée de bactéries. En d'autres termes, l'utilisation d'au moins un agent chélatant pour cultiver des bactéries magnétotactiques aboutit à la production de bactéries magnétotactiques avec un rendement accru par rapport aux procédés connus réalisés sans agent chélatant.

De manière générale, les agents chélatants sont choisis notamment parmi (i) les agents chélatants possédant un ou plusieurs groupes carboxyles, (ii) les agents chélatants possédant un ou plusieurs groupes hydroxyles, (iii) les agents chélatants possédant un ou plusieurs groupes amino et/ou carboxyle et/ou cétone, (iv) les agents chélatants possédant un ou plusieurs groupes phosphonates et/ou acide phosphoniques, (v) les agents chélatants possédant un ou plusieurs groupes bis-phosphonate et/ou tris-phosphonates et/ou tétra-phosphonates, (vi) les agents chélatants possédant un ou plusieurs groupes sulfonates et/ou acides sulfoniques et (vii) les agents chélatants de types polydentés et/ou de types polymériques, par exemple de type polysaccharidique.

On précise qu'un agent chélatant particulier peut comprendre une pluralité de groupes fonctionnels, par exemple parmi les groupes carboxyles, hydroxyles ou amino, et ainsi être classé de manière absolue dans plus d'une catégorie, parmi les catégories (i) à (vii) précitées. Toutefois, les divers agents chélatants peuvent être classés selon le type de groupe fonctionnel principal qui les caractérise, comme spécifié ci-après.

Dans certains modes de réalisation, les agents chélatants sont choisis parmi les agents possédant un ou plusieurs groupes carboxyles, tels que ALA (acide alpha lipoïque), la calcéine, la carboxyfluorescéine, le déferasirox, l'acide dipicolinique, le DTPA (acide diéthylène triamine penta-acétique), l'EDTA (acide éthylène diamine tétra-acétique), l'acide folique (vitamine B9), l'acide lactique, la rhodamine B, un carboxyméthyl-dextrane, l'acide oxalique, l'acide citrique, un composé comprenant un ou plusieurs groupes fonctionnels citrique et/ou citrate, et l'acide phénolique. Un agent chélatant au sens de l'invention peut aussi être un composé comprenant un ou plusieurs groupes fonctionnels acétate et/ou acétique englobant le BAPTA (acide aminophénoxyéthane-tétra-acétique), le CDTA (acide cyclohexane-1,2-diamine tétra-acétique), le EDDHMA (acide éthylène diamine di-(o-hydroxy-p-méthylphényl) acétique), le CaNa2-EDTA, le EDTCA (acide éthylène diamine tétra-acétique avec Cetavlon® (surfactif de type ammonium)), le EDDA (acide éthylène diamine-N,N'-diacétique), le EDDHA (acide éthylènediamine-N,N'-bis(2-hydroxyphényl acétique), le EGTA (acide éthylène glycol-bis-(p-amino-éthyl ether) N,N,N',N'-tétra acétique), le HEDTA (N-(2-hydroxyéthyl)-éthylènediamine triacétique) le HEEDTA (acide hydroxy-2-éthylènediamine triacétique), et le NTA (nitrilo triacétate).

Dans d'autres modes de réalisation, l'agent chélatant est une molécule comprenant un ou plusieurs groupes fonctionnels hydroxyles, tels que le catéchol ou leurs dérivés, ou encore la déferiprone.

Dans d'autres modes de réalisation, l'agent chélatant est une molécule comprenant un ou plusieurs groupes fonctionnels amino, tels que la dopamine et/ou la déferoxamine.

Dans d'autres modes de réalisation, l'agent chélatant est une molécule comprenant un ou plusieurs groupes fonctionnels amino-carboxylique et/ou cétone, tels que la doxorubicine, la caféine, la D-pénicillamine, la pyrroloquinoline, et le HEIDA (acide hydroxyéthylimino-N,N-diéthanoïque).

Dans certains modes de réalisation, l'agent chélatant est une molécule comprenant au moins un groupe fonctionnel phosphonate ou phosphonique, tels que le AEPN (acide 2-aminoéthylphosphonique), le AMP (acide amino-tris-(méthylène-phosphonique)), le ATMP (acide amino tris(méthylène phosphonique)), le CEPA (acide 2-carboxyéthyl phosphonique), le DMMP (diméthyl méthylphosphonate), le DTPMP (acide diéthylènetriamine penta(méthylène phosphonique)), le EDTMP (acide éthylènediamine tétra(méthylène phosphonique)), le HEDP (acide 1-hydroxy éthylidène-1,1-diphosphonique), le HDTMP (acide hexaméthylènediamine tétra (méthylène phosphonique)), le HPAA (acide 2-hydroxyphosphonocarboxylique), le PBTC (acide phosphonobutane-tricarboxylique), le PMIDA (acide N-(phosphonométhyl)iminodiacétique), le TDTMP (acide tétraméthylènediamine tétra(méthylène phosphonique)), le ADP (acide adenosinediphosphorique) ou 1-{12-[4-(difluorure de dipyrrométhènebore)butanoyl]amino}dodecanoyl-2-hydroxy-sn-glycero-3-phosphate, un sel de sodium de l'acide L-α-phosphatidique, et un sel de sodium de 1-palmitoyl-2-(dipyrrométhène boron difluorure) undecanoyl-sn-glycero-3-phospho-L-serine).

Dans certains modes de réalisation, l'agent chélatant est une molécule contenant au moins un groupe fonctionnel bis-phosphonate, tris-phosphonate ou tétra-phosphonate, tels que l'acide 1-hydroxyméthylène-bis-phosphonique, l'acide propanetriphosphonique, l'acide (nitilotris(méthylène))trisphophonique, l'acide (phosphinylidynetris(méthylène)) trisphosphonique. Les acides 1-hydroxyméthylène-bis-phosphoniques englobent l'acide alendronique (commercialisé sous le nom fosamax®), l'acide pamidronique, l'acide zolédronique, l'acide risédronique, l'acide néridronique, l'acide ibandronique (commercialisé sous le nom bondronat®), l'acide minodronique et d'autres composés décrits dans l'état de la technique (voir par exemple L. Wilder et al., J. Med. Chem., 2002, 45, 3721-3728; M. Neves, N. Med. Biol., 2002, 29, 329-338; H. Shinoda et al., Calcif. Tissue Int., 1983, 35, 87-89; M. A. Merrel, Eur. J. Phramacol., 2007, 570, 27-37).

Dans certains modes de réalisation, l'agent chélatant est une molécule comprenant un ou plusieurs groupes fonctionnels sulfonates ou acides sulfoniques, ou encore un groupe dimercapto, tels que le BPDS (bathophénanthrolinedisulfonate ou 4,7-di(4-phénylsulfonate)-1, 10-phénanthroline), le DMPS (Dimercapto-propane sulfonate ou l'acide 2,3-dimercapto-1-propanesulfonique), la sulforhodamine 101, et le DMSA (acide Dimercatptosuccinique).

D'autres exemples d'agents chélatants englobent les ligands polydentés, c'est-à-dire les chélatants ayant plus d'un atome susceptible de se lier à un atome de métal, tels que l'hémoglobine, la chlorophylle, les porphyrines et des composés organiques contenant des cycles pyrrole.

D'autres exemples d'agents chélatants englobent les composés polymériques, en particulier les composés polysaccharidiques.

Ainsi, dans certains modes de réalisation, un agent chélatant est choisi parmi la rhodamine B, l'acide ascorbique, l'acide citrique, l'acide folique, l'érythrosine, l'hémoglobine, un dextran de bas poids moléculaire, l'acide anthranilique, la calcéine, l'alendronate, l'acide 3-[cyclohexylamino]-1-propanesulfonique (CAPS) et l'EDTA. Au sens de l'invention, un dextran de bas poids moléculaire est un dextran ayant un poids moléculaire inférieur à 2000 Da, et préférentiellement inférieur à 1000 Da. Un dextran de bas poids moléculaire a un poids moléculaire supérieur à 100 Da, et préférentiellement supérieur à 500 Da.

Dans certains modes de réalisation, l'agent chélatant est choisi parmi les composés ayant une activité anti-cancéreuse. Un tel agent chélatant peut être par exemple le temozolomide ou certains composés bis-phosphonates.

Le ou les agents chélatants sont avantageusement utilisés dans un milieu de culture de bactéries magnétotactiques à une concentration finale allant de 0,02 µM à 1 mM.

Dans certains modes de réalisation, le ou les agents chélatants sont avantageusement utilisés dans un milieu de culture de bactéries magnétotactiques à une concentration finale allant de 0,02 µM à 500 µM.

Dans certains modes de réalisation, le ou les agents chélatants sont avantageusement utilisés dans un milieu de culture de bactéries magnétotactiques à une concentration finale allant de 0,1 µM à 200 µM.

Les exemples illustrent que la concentration finale optimale de chaque agent chélatant dans le milieu de culture des bactéries magnétotactiques peut varier selon l'agent chélatant qui est choisi. Toutefois, pour chacun des agents chélatants, la concentration finale optimale d'utilisation dans une culture de bactéries magnétotactiques est aisément déterminée par l'homme du métier, par de simples essais de routine en utilisant les conditions de culture décrites dans les exemples.

Les résultats des exemples montrent que les divers agents chélatants testés peuvent être utilisés à une concentration finale allant de 0,04 µM à 40 µM.

Avantageusement, les bactéries magnétotactiques d'intérêt sont mises en culture dans un milieu comprenant au moins une source de fer, telle qu'une solution de quinate de fer, et comprenant des additifs tels que des métaux de transition autres que le fer, et un agent chélatant ou une combinaison d'agents chélatants. A titre illustratif, les bactéries magnétotactiques peuvent être mises en culture dans du milieu MSGM, comme décrit par exemple par Kundu et al. (2010, Indian J Exp Biol, Vol. 48 : 518-5234) ou encore dans les exemples ci-après.

Dans certains modes de réalisation, le ou les agents chélatants sont utilisés pour complexer le fer à la fois sous forme de fer²⁺ et de fer³⁺ dans le rapport molaire de 1 pour 2 de la magnétite, ce qui permet une optimisation de la synthèse des magnétites des magnétosomes.

Dans certains modes de réalisation, le ou les agents chélatants sont utilisés à des pH différents de l'optimum publié pour la culture des souches de bactéries magnétotactiques ce qui permet une optimisation de la synthèse des magnétites des magnétosomes.

En général, un procédé de culture de bactéries magnétotactiques conforme à la présente description comprend les étapes suivantes :
a) addition de bactéries magnétotactiques dans un milieu de culture approprié contenant un agent chélatant ou une combinaison d'agents chélatants,
b) culture des bactéries magnétotactiques dans ledit milieu, dans des conditions opératoires choisies,
c) récupération des cellules de bactéries magnétotactiques obtenues à la fin de l'étape b).

En général, l'étape b) est réalisée à la température ambiante, c'est-à-dire à environ 25 °C.

En général, on poursuit l'étape b) jusqu'à atteindre au moins la phase de croissance exponentielle des bactéries magnétotactiques. Dans certains modes de réalisation, l'étape b) est poursuivie jusqu'à atteindre la phase de croissance stationnaire des bactéries magnétotactiques.

Dans certains modes de réalisation, la durée de l'étape b) va de 1 à 15 jours suivant l'ensemencement des bactéries dans le milieu de culture. La durée de l'étape b) est avantageusement de 2 à 9 jours, et préférentiellement de 2 à 6 jours.

Puis, à l'étape c), la récupération des bactéries magnétotactiques peut être réalisée de manière classique, par exemple par centrifugation de la suspension de cellules bactériennes, puis récupération du culot contenant les bactéries magnétotactiques.

Dans des modes de réalisation préférés, le procédé de culture de bactéries magnétotactiques décrit ci-dessus consiste en un procédé de culture en continu.

On peut réaliser le procédé de culture en continu à l'aide de tout type connu de dispositif de culture cellulaire en continu. A titre illustratif, l'homme du métier peut utiliser un dispositif Biostat®Aplus commercialisé par la société Sartorius (France).

Typiquement, un tel dispositif comprend :
(I) Une enceinte de culture régulée en température, pH, pression d'oxygène,
(II) Un moyen pour introduire, préférentiellement en continu, du milieu stérile de culture et/ou des bactéries dans l'enceinte de culture,
(III) Un moyen pour récupérer, préférentiellement en continu, du milieu de culture et/ou tout ou partie des bactéries cultivées dans l'enceinte de culture,

Préférentiellement, l'enceinte de culture (I) comprend aussi un ou plusieurs des moyens suivants :
(1) un moyen pour réguler la température de la suspension bactérienne présente à l'intérieur de l'enceinte. Un tel moyen peut consister en un échangeur thermique disposé sur la paroi externe de l'enceinte de culture, tel qu'un échangeur à circulation d'eau ;
(2) un dispositif d'agitation de la suspension de cellules bactériennes contenue dans l'enceinte de culture ;
(3) une ou plusieurs sondes de mesure de paramètres physiques et/ou chimiques des conditions de culture, ce qui englobe (a) des sondes de mesure des gaz dissous dans le milieu de suspension des cellules bactériennes, telles qu'une sonde de mesure de l'oxygène dissous et/ou une sonde de mesure de l'azote dissous, (b) une sonde de mesure de pH, ou encore (c) une sonde de mesure de la température.

Dans certains modes de réalisation d'un procédé de culture réalisé en continu, (a) on alimente l'enceinte de culture en milieu de culture stérile frais, (b) on ensemence ledit milieu de culture avec la quantité désirée de départ de bactéries magnétotactiques, (c) on cultive lesdites bactéries pendant une durée suffisante pour leur croissance, et (d) on prélève une partie de la suspension cellulaire à des intervalles de temps choisis, étant entendu que (e) la culture des bactéries est poursuivie dans l'enceinte de culture après chaque étape de prélèvement partiel de la suspension bactérienne.

Dans certains modes de réalisation d'un procédé de culture en continu, les étapes (d) et (e) peuvent être réitérées un nombre de fois allant de 2 à 1000 fois, par exemple de 1 à 100 fois, ou dans certains cas de 1 à 10 fois.

Dans certains modes de réalisation, on procède ensuite à l'extraction des chaînes de magnétosomes à partir des cellules qui sont récoltées à la fin du procédé de culture proprement dit. A titre illustratif, les chaînes de magnétosomes peuvent être extraites selon un procédé comprenant les étapes suivantes :
(i) lyse des cellules récoltées à la fin de leur culture, par exemple par sonication, afin d'obtenir un liquide contenant des chaînes de magnétosomes et des débris cellulaires en suspension,
(ii) séparation des chaînes de magnétosomes et des débris cellulaires en appliquant un champ magnétique à la solution obtenue à la fin de l'étape (i), par exemple à l'aide d'un aimant puissant (p. ex. un aimant ayant une puissance allant de 0,1 à 1 T),
(iii) récupération des chaînes de magnétosomes séparées, par exemple par élimination du surnageant contenant les débris cellulaires et récupération d'un culot de magnétosomes.

En général, les chaînes de magnétosomes sont récupérées sous la forme d'un culot puis sont remises en suspension dans un tampon approprié pour leur utilisation future, comme cela est illustré dans les exemples. Préférentiellement, on utilise un milieu tampon non salin.

Dans certains modes de réalisation, les chaînes de magnétosomes peuvent être sélectionnées selon leur taille, par exemple en soumettant celles-ci à des champs magnétiques de puissance croissante (p. ex. des champs magnétiques ayant une puissance allant de 0,05 à 1 T), ou bien en soumettant celles-ci à une étape de chromatographie d'exclusion de taille.

Les chaînes de magnétosomes obtenues à partir des bactéries magnétotactiques cultivées en présence d'au moins un agent chélatant sont utiles notamment pour le traitement de cancers par thermothérapie. Ainsi, les chaînes de magnétosomes obtenues par culture des bactéries magnétotactiques en présence d'au moins un agent chélatant peuvent être utilisées *in vivo* pour détruire des cellules tumorales, du fait d'une génération locale de chaleur lorsque lesdites chaînes de magnétosomes sont soumises à un champ magnétique alternatif.

### EXEMPLES

### Exemple 1 : Culture des bactéries magnétotactiques et extraction des magnétosomes

### 1.1. Bactéries

On a utilisé des bactéries magnétotactiques de l'espèce *Magnetospirillum magneticum,* souche AMB-1, disponibles auprès de l'ATCC sous la référence ATTC n° 700274.

Les cellules ont été mises en culture dans des conditions micro-anaérobie, c'est-à-dire dans un milieu de culture qui n'a pas été dégazé mais qui est clos et sans contact avec l'oxygène. La culture des bactéries s'est faite à la température du laboratoire (environ 25°C) dans une culture liquide dans un milieu MSGM légèrement modifié (milieu ATCC 1653), dont la constitution est décrite ci-après.

### 1.2. Milieu de culture témoin

Pour un volume d'un litre, le milieu de culture contient 0,68 g de phosphate de potassium monobasique, 0,85 g de succinate de sodium, 0,57 g de tartrate de sodium, 0,083 g d'acétate de sodium, 225 µl de resazurine à 0,2 %, 0,17 g de nitrate de sodium, 0,04 g d'acide L-ascorbique, 2 ml d'une solution à 10 mM de quinate de fer, 10 ml d'une solution de vitamines de Woolf et 5 ml d'une solution de minéraux de Woolf.

La solution de quinate de fer a été préparée en dissolvant 0,19 g d'acide quinique et 0,29g de FeCl₃.6H₂O dans 100 millilitres d'eau distillée.

La solution de minéraux de Woolf contient, dans 1 litre d'eau distillée, 0,5 g d'acide nitrilotriacétique, (NTA, C₆H₉NO₆), 1,5 g de sulfate de magnésium HEPTA (MgSO₄.7H₂O), 1 g de chlorure de sodium, 0,5 g de sulfate de manganèse (MnSO₄.H₂O), 100 mg de sulfate de fer heptahydrate (FeSO₄.7H₂O), 100 mg de nitrate de cobalt (CO(NO₃)₂.7H₂0), 100 mg de chlorure de calcium (CaCl₂), 100 mg de sulfate de zinc heptahydrate (ZnSO₄.7H₂O), 10 mg de sulfate de cuivre pentahydrate (CuSO₄.5H₂O), 10 mg de sulfate d'aluminium et de potassium dodécahydrate (AlK(SO₄).12H₂O), 10 mg d'acide borique (H₃BO₃), 10 mg de molybdate de sodium (Na₂MoO₄.2H₂O), 2 mg de sélénite de sodium (Na₂SeO₃), 10 mg de tungstate de sodium dihydrate (Na₂WO₄.2H₂0) et 20 mg de chlorure de nickel (NiCl₂.6H₂O).

La solution de vitamines de Woolf a été préparée en dissolvant, dans un litre d'eau distillée, 2,2 mg d'acide folique (vitamine B9), 10,2 mg de pyridoxine (vitamine B6), 5,2 mg de riboflavine (vitamine B2), 2,2 mg de biotine (vitamine H ou B7), 5,2 mg de thiamine (vitamine B1), 5,2 mg d'acide nicotinique (vitamine B3 ou PP), 5,2 mg d'acide pantothénique (vitamine B5), 0,4 mg de vitamine B12, 5,2 mg d'acide amino-benzoïque, 5,2 mg d'acide thiotique et 900 mg de phosphate de potassium.

Le pH du milieu a été ajusté à 6,85 en utilisant une solution d'hydroxyde de sodium à 5 M.

### 1.3 Milieu de culture contenant les agents chélatants du fer :

Afin de préparer le milieu de culture contenant les différents agents chélatants du fer, ceux-ci ont été introduits à différentes concentrations au milieu de culture décrit ci-dessus (paragraphe 1.2).

### 1.4. Extraction des chaînes de magnétosomes

Les cellules ont été récoltées durant la phase stationnaire. La phase stationnaire survient lorsque le milieu est complètement réduit, comme indiqué par un changement de coloration, du rose vers l'incolore.

Les cellules sont récoltées durant la phase stationnaire par centrifugation à 4000 g pendant 20 minutes. Le surnageant est éliminé et les cellules sont remises en suspension dans 3 ml d'eau déionisée.

Pour extraire les chaînes de magnétosomes, 1 ml de suspension cellulaire obtenue comme décrit ci-dessus est à nouveau centrifugé puis remis en suspension dans un tampon Tris-HCl 10 mM à pH 7,4 puis la suspension cellulaire est soumise à une étape de sonication pendant 120 minutes à la puissance de 30 W, afin de lyser les cellules et libérer les chaînes de magnétosomes. Des durées de sonication de 60 et 180 minutes peuvent également être utilisées. Pour une durée de sonication inférieure à 60 minutes, les bactéries ne sont pas toutes lysées. Pour une durée de sonication supérieure à 180 minutes, on observe un début d'aggrégation, du fait de la présence de magnétosomes individuels qui sont agrégés.

Après sonication, la suspension de chaînes de magnétosomes est séparée en plaçant un puissant aimant de neodynium (0,1 - 1 T) à proximité du tube, puis le matériau magnétique est récupéré sous forme d'un culot.

Le surnageant contenant les débris cellulaires et autres molécules organiques est éliminé.

Les chaînes de magnétosomes sont lavées 10 à 20 fois dans de l'eau déionisée à pH 7,4 puis sont remises en suspension dans de l'eau stérile déionisée.

### 1.5. Mesure des propriétés magnétiques:

Nous avons mesuré la concentration en maghémite d'une suspension de chaînes de magnétosomes extraites des bactéries magnétotactiques à partir de la mesure de l'absorption à 480 nm de cette suspension. La méthode de calibration entre la valeur de l'absorption et la quantité de maghémite est décrite ailleurs (PCT/EP2010/067765). Ces mesures ont été réalisées afin de déterminer la quantité de chaînes de magnétosomes produites par les bactéries magnétotactiques après un temps de croissance donné. Les quantités de chaînes de magnétosomes produites en absence et en présence des agents chélatants ont ensuite été comparées.

Nous avons également mesuré le moment magnétique de différentes suspensions de bactéries magnétotactiques déposée sur un buvard sous application d'un champ magnétique de 500 Oe ou de 1000 Oe. Le moment magnétique a été mesuré pour 10 ml de suspension de bactéries magnétotactiques récoltées à différents jours de croissance, centrifugés puis re-suspendus dans 100 µl d'eau distillée. Ces 100 µl contenant les bactéries magnétotactiques sont ensuite redéposés sur du papier absorbant non magnétique qui lui-même est inséré dans une capsule que l'on positionne dans le magnétomètre pour effectuer les mesures magnétiques. La valeur du moment magnétique étant proportionnelle à la quantité de magnétosomes présente dans l'échantillon, sa mesure indique la présence ou non de magnétosomes. Lorsqu'on mesure un moment magnétique nul, cela indique que la quantité de magnétosomes présente dans l'échantillon est tellement faible (ou nulle) qu'elle n'est pas détectable à l'aide du magnétomètre (un SQUID très sensible).

### Exemple 2 : Influence de la présence d'agents chélatants dans le milieu de culture sur la croissance bactérienne et le nombre de magnétosomes, mesurée au bout de 7 jours de culture.

Dans cet exemple, les bactéries magnétotactiques ont été cultivées en présence de différents agents chélatants et récoltées au bout de 7 jours. L'absorption et la concentration en oxyde de fer des chaînes de magnétosomes extraites des bactéries ont ensuite été mesurées.

Comme le montrent les Figures 1(a), 1(c), 2(a), 2(c), les valeurs de l'absorption mesurées à 565 nm des suspensions de bactéries magnétotactiques cultivées en présence d'agents chélatants sont supérieures à celles des suspensions de bactéries cultivées en absence d'agents chélatants et ce pour un grand nombre d'agents chélatants et de concentrations testées. Il s'agit de l'hémoglobine de concentrations 0,4 µM, 4 µM et 40 µM (Fig. 1(a)), l'EDTA de concentrations 0,4 µM, 4 µM et 40 µM (Fig. 1(a)), l'acide anthranilique de concentration 0,4 µM (Fig. 1(a)), l'acide citrique de concentrations 4 µM et 40 µM (Fig. 1(c)), l'acide 3(N-morpholino)propanesulfonique de concentrations 4 µM et 40 µM (Fig. 1(c)), l'acide 3-[Cyclohexylamino]-1-propanesulfonique de concentrations 0,4 µM, 4 µM et 40 µM (Fig. 1(c)), le néridronate de concentration 40 µM (Fig. 2(a)), le nicotinamide de concentration 40 µM (Fig. 2(a)), la Rhodamine B de contrations 0,4 µM, 4 µM et 40 µM (Fig. 2(c)), la calcéine de concentration 4 µM et 40 µM (Fig. 2(c)) et le dextrane de concentration 40 µM (Fig. 2(c)). Les effets les plus prononcés sont observés pour l'hémoglobine de concentration 40 µM, l'acide citrique de concentration 4 µM, le nicotinamide de concentration 40 µM et la calcéine de concentration 40 µM pour lesquels l'absorption est de 1.5 à 2 fois supérieur à celle des bactéries témoin.

Les Figures 1(b), 1(d), 2(b) et 2(d) montrent la concentration en oxyde de fer, proportionnelle à la concentration en magnétosomes, de différentes suspensions de chaînes de magnétosomes extraites des bactéries pour des bactéries magnétotactiques récoltées après 7 jours de culture et cultivées en présence (ou non) d'une variété d'agents chélatants. La production de magnétosomes est accrue par rapport aux bactéries témoin pour une variété d'agents chélatants différents introduits dans le milieu de culture à différentes concentrations. Cette augmentation de la production en magnétosomes a lieu lorsque l'on introduit dans le milieu de culture des bactéries magnétotactiques de l'hémoglobine à concentrations 0,4 µM, 4 µM, 40 µM (Fig. 1(b)), de l'EDTA à concentrations 0,4 µM et 4 µM (Fig. 1(b)), de l'acide anthranilique à concentration 0,4 µM (Fig. 1(b)), de l'acide citrique à concentration 4 µM et 40 µM (Fig. 1(d)), de l'acide 3-(N-morpholino)propanesulfonique à 0,4 µM (Fig. 1(d)), de l'acide 3-(Cyclohexylamino)-1-propanesulfonique à concentrations 0,4 µM, 4 µM, 40 µM (Fig. 1(d)), de l'alendronate à concentrations 0,4 µM, 4 µM et 40 µM (Fig. 2(b)), du Dextran à concentration 40 µM (Fig. 2(d)), de la calcéine à concentrations 4 µM et 40 µM (Fig. 2(d)) et de la Rhodamine B à concentrations 0,4 µM, 4 µM et 40 µM. Les effets les plus prononcés sont observés avec l'hémoglobine pour laquelle la production en oxyde de fer est augmentée d'un facteur 7-8 pour une concentration en hémoglobine dans le milieu de culture de 0,4 µM ou 4 µM (Fig. 1(b)).

On peut tirer de cet exemple les conclusions suivantes :
(i) Le nombre de bactéries et la production de magnétosomes sont tous deux plus importants en présence d'agents chélatants qu'en absence d'agents chélatants et ce pour un grand nombre d'agents chélatants et de concentrations testées.
(ii) La concentration de 400 µM est la concentration maximale en agents chélatants au-delà de laquelle ni la croissance bactérienne ni la production de magnétosomes ne sont stimulées (Figs. 1 et 2).
(iii) La production en magnétosomes augmente en général plus que le nombre de bactéries en présence d'agents chélatants (cf. l'exemple de l'hémoglobine). Cela suggère qu'en présence d'agents chélatants la stimulation de la croissance bactérienne s'accompagne soit d'un nombre accru de magnétosomes par bactérie magnétotactique soit d'un pourcentage plus faible de bactéries ne produisant pas de magnétosomes.

### Exemple 3 : Effets d'agents chélatants sur la croissance bactérienne

### A. Matériels et méthodes

On a préparé des cultures de *Magnetospirillum magneticum* comme décrit à l'exemple 1. Les bactéries ont été cultivées dans des tubes de 10 millilitres. On a inoculé au temps «J0» (Jour 0) un volume de 100 microlitres de milieu de culture avec 0.1 10⁶ cellules bactériennes. Les cultures en suspension ont été placées à température ambiante (environ 25°C) et la croissance des bactéries au cours du temps a été suivie, par mesure de D. O. à la longueur d'onde de 565 nanomètres.

On a préparé les séries d'échantillons de culture suivantes :
- des échantillons de culture témoin, comprenant les cellules bactériennes dans le milieu de culture exempt d'agent chélatant ;
- une série d'échantillons de culture contenant l'agent chélatant Rhodamine B, aux concentrations croissantes de 0,4 µM, 4 µM, 40 µM et 400 µM ;
- une série d'échantillons de culture contenant l'agent chélatant acide ascorbique, aux concentrations croissantes de 0,4 µM, 4 µM, 40 µM et 400 µM ;
- une série d'échantillons de culture contenant l'agent chélatant érythrosine, aux concentrations croissantes de 0,4 µM, 4 µM, 40 µM et 400 µM ;
- une série d'échantillons de culture contenant l'agent chélatant acide folique, aux concentrations croissantes de 0,4 µM, 4 µM, 40 µM et 400 µM ;

Plusieurs séries d'essais ont été réalisées, dont les résultats sont reportés sur les figures 3 à 10. Les bactéries ont été cultivées pendant 12 jours en tube et on a mesuré l'absorbance sur la totalité du spectre allant de 400 à 700 nanomètres, respectivement (i) au deuxième jour (J2) après l'ensemencement, (ii) au troisième jour (J3) après l'ensemencement, (iii) au quatrième jour (J4) après l'ensemencement, (iv) au septième jour (J7) après l'ensemencement, (v) au huitième jour (J8) après l'ensemencement et (vi) au neuvième jour (J9) après l'ensemencement.. Les moments magnétiques de différentes suspensions de bactéries magnétotactiques déposées sur un buvard ont été mesurés aux jours J7, J8 et J9.

### B. Résultats

Les résultats des figures 3(a) à 3(f) montrent que la rhodamine B stimule la croissance des bactéries magnétotactiques, comme cela est observé par l'augmentation d'absorbance des suspensions cellulaires cultivées en présence de cet agent chélatant, par rapport aux suspensions cellulaires cultivées en l'absence de Rhodamine B. La stimulation de la croissance bactérienne par la rhodamine B dépend de la concentration finale en agent chélatant, un maximum de stimulation de croissance étant observé dans cet essai pour une concentration finale de 0,4 µM à 40 µM. La concentration de 400 µM est plutôt défavorable à la croissance cellulaire, cet effet pouvant être dû à une certaine cytotoxicité de la rhodamine B, à forte concentration. Les effets de la rhodamine B sont les plus importants pendant les six premiers jours de culture, c'est-à-dire pendant la phase de croissance exponentielle. La Figure 3(c) montre que l'effet le plus important est observé au quatrième jour de culture (J4). Les variations durant les 7 premiers jours de croissance de l'absorption en fonction du jour de croissance des suspensions contenant des bactéries magnétotactiques cultivées en présence de Rhodamine ont été fitées (courbes ajustées) par des polynômes (Fig. 4(a)). Les courbes de croissance pour les bactéries cultivées en présence de rhodamine B sont au-dessus de celles des bactéries cultivées en absence de rhodamine B (sauf pour la concentration en rhodamine B de 400 µM, Fig. 4(a)). Ceci confirme l'effet stimulant de la rhodamine B sur la croissance bactérienne. Les moments magnétiques des bactéries ont également été mesurés aux jours J7, J8 et J9 sous application d'un champ magnétique de 500 Oe (Fig. 4(b)) ou 1000 Oe (Fig. 4(c)). Les Figures 4(b) et 4(c) montrent l'apparition plus précoce, c'est à dire à J7, du moment magnétique pour les bactéries cultivées en présence de 0,4 µM ou 4 µM rhodamine B que pour celles cultivées en absence de rhodamine B où le moment magnétique n'est mesurable qu'à J9. Ces résultats indiquent qu'en présence de 0,4 µM ou 4 µM de rhodamine B, les magnétosomes sont synthétisés plus rapidement par les bactéries magnétotactiques qu'en absence de rhodamine B.

Les résultats des figures 5(a) à 5(f) montrent que l'acide ascorbique stimule la croissance des bactéries magnétotactiques, comme cela est observé par l'augmentation d'absorbance des suspensions cellulaires cultivées en présence de cet agent chélatant, par rapport aux suspensions cellulaires cultivées en l'absence d'acide ascorbique. La stimulation de la croissance bactérienne par l'acide ascorbique dépend de la concentration finale en agent chélatant, un maximum de stimulation de croissance étant observé dans cet essai pour une concentration finale de 0,4 µM à 40 µM. La concentration de 400 µM est plutôt défavorable à la croissance cellulaire, cet effet pouvant être dû à une légère toxicité de l'acide ascorbique, à forte concentration. Les effets de l'acide ascorbique sont les plus importants pendant les quatre premiers jours de culture, c'est-à-dire pendant la phase de croissance exponentielle. L'effet le plus prononcé est observé pour 0,4 µM d'acide ascorbique après 4 jours de culture (Fig. 5(c)). Les variations de l'absorption durant les 7 premiers jours de croissance en fonction du jour de croissance des suspensions contenant des bactéries magnétotactiques cultivées en présence d'acide ascorbique ont été fitées (courbes ajustées) par des polynômes (Fig. 6(a)). Les courbes de croissance pour les bactéries cultivées en présence d'acide ascorbique sont globalement au-dessus de celles des bactéries cultivées en absence d'acide ascorbique (sauf pour la concentration en acide ascorbique de 400 µM) (Fig. 6(a)). Ceci confirme l'effet stimulant de l'acide ascorbique sur la croissance bactérienne. Les moments magnétiques des bactéries ont également été mesurés aux jours J7, J8 et J9 sous application d'un champ magnétique de 500 Oe (Fig. 6(b)) ou 1000 Oe (Fig. 6(c)). Les Figures 6(b) et 6(c) montrent l'apparition plus précoce, c'est à dire à J7, du moment magnétique pour les bactéries cultivées en présence de 0,4 µM, 4 µM 40 µM ou 400 µM d'acide ascorbique qu'en absence d'acide ascorbique où le moment magnétique n'est mesurable qu'à J9 (Figs. 6(b) et 6(c)). Ces résultats indiquent qu'en présence de 0,4 µM, 4 µM, 40 µM ou 400 µM d'acide ascorbique, les magnétosomes sont synthétisés plus rapidement par les bactéries magnétotactiques qu'en absence d'acide ascorbique. Comparé à la rhodamine B, l'effet de l'acide ascorbique sur la croissance bactérienne est similaire, mais la production de magnétosomes est stimulée pour une gamme de concentrations plus large.

Les résultats des figures 7(a) à 7(f) et de la figure 8 montrent que l'érythrosine stimule moins la croissance des bactéries magnétotactiques que la rhodamine B ou l'acide ascorbique. Une absorption plus importante des bactéries magnétotactiques cultivées en présence de 0,4 µM ou 4 µM d'érythrosine est observée au jour J4. Pour les autres jours de cultures et les autres concentrations, l'absorption des bactéries cultivées en présence d'érythrosine est soit similaire soit plus faible que l'absorption des bactéries témoin. Cela est peut-être du à la présence de l'iode dans l'érythrosine qui serait toxique pour les bactéries.

Les résultats des figures 9(a) à 9(f) et de la figure 10 montrent que l'acide folique stimule la croissance des bactéries magnétotactique soit en permettant aux bactéries de se développer plus rapidement pendant les deux premiers jours de croissance (acide folique de concentration 4 µM, Figure 10) soit en permettant aux bactéries de se multiplier en un plus grand nombre durant les 4 premiers jours de croissance (acide folique de concentrations 0,4 µM ou 40 µM).

On peut tirer de cet exemple les conclusions suivantes :
(i) Les bactéries magnétotactiques se développent et produisent des magnétosomes plus rapidement pour trois des agents chélatants testés (Rhodamine, acide ascorbique et acide folique) à des concentrations variant entre 0,4 µM et 40 µM suivant les agents chélatants.
(ii) Les agents chélatants stimulent non seulement la croissance bactérienne mais aussi la production de magnétosomes.

### Exemple 4 : Culture des bactéries magnétotactiques en flux continu.

La stimulation de la croissance bactérienne et de la production de magnétosomes permet d'envisager un système de culture de bactéries magnétotactiques en flux continu. Pour ce faire, on peut utiliser un dispositif expérimental du type Biostat®Aplus commercialisé par la société Sartorius. Suivant ce dispositif, le milieu de culture des bactéries contenant les agents chélatants est introduit en continu dans un Biostat où les bactéries se développent dans des conditions contrôlées (contrôle de la température, du pH, de la concentration en oxygène). Les bactéries qui se sont développées sont ensuite récupérées en continu dans une bouteille. Ce dispositif fonctionne à l'aide d'un système de pompes qui permet de faire circuler le milieu de culture et les bactéries.

Comme les bactéries se développent plus rapidement en présence d'agents chélatants qu'en absence d'agents chélatants, ce dispositif permet de cultiver plus de bactéries en présence qu'en absence d'agents chélatants.

## Revendications

1. Utilisation d'au moins un agent consistant en un agent chélatant pour stimuler la croissance de bactéries magnétotactiques.

2. Utilisation selon la revendication 1, **caractérisée en ce que** ledit agent chélatant est un agent chélatant du fer.

3. Utilisation selon la revendication 1, **caractérisée en ce que** ledit agent chélatant est choisi parmi (i) les agents chélatants possédant un ou plusieurs groupes carboxyles, (ii) les agents chélatants possédant un ou plusieurs groupes hydroxyles, (iii) les agents chélatants possédant un ou plusieurs groupes amino et/ou carboxyle et/ou cétone, (iv) les agents chélatants possédant un ou plusieurs groupes phosphonate et/ou acide phosphonique, (v) les agents chélatants possédant un ou plusieurs groupes bis-phosphonate et/ou tris-phosphonate et/ou tetra-phosphonate, (vi) les agents chélatants possédant un ou plusieurs groupes sulfonate et/ou acide sulfonique et (vii) les agents chélatants de type polydentés.

4. Utilisation selon l'une des revendications 1 à 3, **caractérisée en ce que** ledit agent chélatant est choisi parmi la rhodamine B, l'acide ascorbique, l'acide citrique, l'acide folique, l'érythrosine, l'hémoglobine, un dextran de bas poids moléculaire, l'acide anthranilique, la calcéine, l'alendronate, l'acide 3-[cyclohexylamino]-1-propanesulfonique (CAPS) et l'EDTA.

5. Utilisation selon l'une des revendications 1 à 4, **caractérisée en ce que** le ou les agents chélatants sont utilisés dans le milieu de culture de bactéries magnétotactiques à une concentration finale allant de 0,02 µM à 1 mM, avantageusement de 0,02 µM à 500 µM, et préférentiellement de 0, 1 µM à 200 µM.

6. Utilisation selon la revendication 5, **caractérisée en ce que** le ou les agents chélatants sont utilisés dans le milieu de culture de bactéries magnétotactiques à une concentration finale allant de 0,4 µM à 40 µM.

## Patentansprüche

1. Verwendung eines aus mindestens aus einem Chelatbildner bestehenden Mittels zur Stimulation des Wachstums von magnetotaktischen Bakterien.

2. Verwendung nach Anspruch 1,
**dadurch gekennzeichnet, dass** der Chelatbildner ein Eisenchelatbildner ist.

3. Verwendung nach Anspruch 1,
**dadurch gekennzeichnet, dass** der Chelatbildner ausgewählt ist aus (i) Chelatbildnern mit einer oder mehreren Carboxylgruppen, (ii) Chelatbildnern mit einer oder mehreren Hydroxylgruppen, (iii) Chelatbildnern mit einer oder mehreren Amino- und / oder Carboxyl- und / oder Ketongruppen, (iv) Chelatbildnern mit einer oder mehreren Phosphonat- und / oder Phosphonsäuregruppen, (v) Chelatbildnern mit einer oder mehreren Bisphosphonat- und/ der Triphosphonat- und / oder Tetraphosphonatgruppen, (vi) Chelatbildner mit einer oder mehreren Sulfonat- und/oder Sulfonsäuregruppen und (vii) Chelatbildnern vom Polydentat-Typ.

4. Verwendung nach einem der Ansprüche 1 bis 3,
**dadurch gekennzeichnet, dass** der Chelatbildner ausgewählt ist aus Rhodamin B, Ascorbinsäure, Zitronensäure, Folsäure, Erythrosin, Hämoglobin, ein Dextran mit niedrigem Molekulargewicht, Anthranilsäure, Calcein, Alendronat, 3-[Cyclohexylamino] -1-propansulfonsäure (CAPS) und EDTA.

5. Verwendung nach einem der Ansprüche 1 bis 4,
**dadurch gekennzeichnet, dass** der oder die Chelatbildner im Kulturmedium von magnetotaktischen Bakterien in einer Endkonzentration im Bereich von 0,02 µM bis 1 mM, vorteilhaft von 0,02 µM bis 500 µM und vorzugsweise von 0,1 µM bis 200 µM verwendet werden.

6. Verwendung nach Anspruch 5,
**dadurch gekennzeichnet, dass** der oder die Chelatbildner im Kulturmedium von magnetotaktischen Bakterien in einer Endkonzentration im Bereich von 0,4 µM bis 40 µM verwendet werden.

## Claims

1. The use of at least one agent consisting of a chelating agent for stimulating the growth of magnetotactic bacteria.

2. The use as claimed in claim 1, **characterized in that** said chelating agent is an iron-chelating agent.

3. The use as claimed in claim 1, **characterized in that** said chelating agent is chosen from (i) chelating agents which have one or more carboxyl groups, (ii) chelating agents which have one or more hydroxyl groups, (iii) chelating agents which have one or more amino and/or carboxyl and/or ketone groups, (iv) chelating agents which have one or more phosphonate and/or phosphonic acid groups, (v) chelating agents which have one or more bisphosphonate and/or trisphosphonate and/or tetraphosphonate groups, (vi) chelating agents which have one or more sulfonate and/or sulfonic acid groups, and (vii) chelating agents of polydentate type.

4. The use as claimed in one of claims 1 to 3, **characterized in that** said chelating agent is chosen from rhodamine B, ascorbic acid, citric acid, folic acid, erythrosine, hemoglobin, a low-molecular-weight dextran, anthranilic acid, calcein, alendronate, 3-[cyclohexylamino]-1-propanesulfonic acid (CAPS) and EDTA.

5. The use as claimed in one of claims 1 to 4, **characterized in that** the chelating agent(s) is (are) used in the culture medium for magnetotactic bacteria at a final concentration ranging from 0.02 µM to 1 mM, advantageously from 0.02 µM to 500 µM, and preferentially from 0.1 µM to 200 µM.

6. The use as claimed in claim 5, **characterized in that** the chelating agent(s) is (are) used in the culture medium for magnetotactic bacteria at a final concentration ranging from 0.4 µM to 40 µM.
